# EUROPÄISCHE PATENTSCHRIFT

(11) **EP 0 965 316 B1**
(45) Veröffentlichungstag und Bekanntmachung des Hinweises auf die Patenterteilung: **08.12.2004**
(21) Anmeldenummer: 99109837.7
(22) Anmeldetag: 19.05.1999
(51) Int. Cl.: A61F 13/15

(54) **Tampon und Verfahren zum Verpacken**
Tampon and process for wrapping
Tampon et procédé d'emballage

(30) Priorität: 15.06.1998 DE 19826541
(43) Veröffentlichungstag der Anmeldung: 22.12.1999
(73) Patentinhaber: Ruggli Projects AG, 6332 Hagendorn Cham (CH)
(72) Erfinder: Kuhn, Kurt, 8175 Windlach (CH)
(74) Vertreter: Christophersen, Ulrich Rudolf

(56) Entgegenhaltungen:
- WO-A-84/02840
- DE-A- 3 634 704
- US-A- 2 587 515

## Beschreibung

Die Erfindung betrifft einen Tampon, insbesondere für die Frauenhygiene, bestehend aus einem im wesentlichen zylindrischen Grundkörper mit einem in Einführrichtung vorderen Ende, das abgerundet oder kegelig geformt ist, sowie einem hinteren Ende mit einer Stirnfläche, aus der ein mit einem Teil seiner Länge in dem Grundkörper befestigter Faden oder ein Bändchen herausgeführt ist, wobei Grundkörper und Faden von einer zur Benutzung des Tampons entfernbaren Schutzhülle umschlossen sind. Die Erfindung bezieht sich außerdem auf ein Verfahren zum Verpacken eines Tampons.

Bekannte Tampons bestehen aus einem Faservliesmaterial, das mittels geeigneten Preßwerkzeugen in die voranstehend beschriebene Form gebracht wird. Die bekannten Tampons unterscheiden sich vor allem hinsichtlich ihrer Formsteifigkeit und Saugleistung. Letzteres wird durch die von dem Tampon bewirkte Sauggeschwindigkeit sowie Flüssigkeitsaufnahmekapazität bestimmt. Eine gleichmäßige und hohe Flüssigkeitsaufnahme ist dabei von der wirksamen Oberfläche des Tampons abhängig. Um diese Absorptionsfähigkeit des Tampons nach der Herstellung nicht durch Lagerung und Transport zu beeinträchtigen, ist die gewöhnlich aus Cellophan gefertigte Schutzhülle vorgesehen.

Im WO-A-84/02 840 wird ein Tampon beschrieben, dessen Rückholband am rückwärtigen Verschluß der Verpackungsfolie befestigt ist. Die Verbindungsart wird nicht näher beschrieben.

Die bekannten Tampons dieser Art weisen den Nachteil auf, daß der zum Rückholen dienende Faden beim Umschließen des Tampons mit der weitgehend luft- und feuchtigkeitsdicht abschließenden Schutzhülle in die Faserstruktur des Tampons eingedrückt wird. Da ein Tampon üblicherweise nur mit ausgestrecktem Faden verwendet wird, ist es erforderlich, den in die Faserstruktur eingedrückten Faden vor dem Gebrauch herauszulösen. Dies birgt nicht nur die Gefahr einer Beschädigung der Faserstruktur, etwa durch Fingernägel, und damit einer Beeinträchtigung der Saugleistung des Tampons, sondern ist auch unter hygienischen Gesichtspunkten unbefriedigend.

Davon ausgehend liegt der vorliegenden Erfindung die **Aufgabe** zu Grunde, einen Tampon mit bei der Anwendung verbesserter Handhabung zu schaffen und zudem ein Verfahren zum Verpacken eines solchen Tampons anzugeben.

Diese Aufgabe ist bei einem Tampon mit den voranstehend genannten Merkmalen erfindungsgemäß dadurch **gelöst**, daß der aus dem Grundkörper herausgeführte Teil des Fadens bei von der Schutzhülle umschlossenem Tampon vollständig im Bereich der hinteren Stirnfläche angeordnet ist, wobei ein an dem die hintere Stirnfläche des Tampons abdeckenden Teil der Schutzhülle anliegendes Teilstück des Fadens stoffschlüssig mit der Schutzhülle verbunden ist.

Bei einem solchermaßen ausgebildeten Tampon ist es entbehrlich, den Faden direkt zu ergreifen, um ihn vor Gebrauch auszustrecken. Ursächlich hierfür ist, daß der aus dem Grundkörper herausgeführte Teil des Fadens beim Abziehen des die hintere Stirnfläche abdeckenden Teils der Schutzhülle gleichzeitig vom Tampon gelöst und gestreckt wird. Dies ist darauf zurückzuführen, daß sich der Faden außerhalb des Grundkörpers bei noch nicht entfernter Schutzhülle ausschließlich im Bereich der hinteren Stirnfläche befindet und lediglich zur Sicherung seiner Lage locker mit dem Tampon verbunden ist. Beim Abziehen des die hintere Stirnfläche abdeckenden Teils der Schutzhülle löst sich daher der aus dem Grundkörper herausgeführte Teil des Fadens zwangsläufig von dem Tampon, da das an der Schutzhülle anliegende Teilstück des Fadens stoffschlüssig mit dieser verbunden ist. Der Faden wird also gestreckt, wobei erst bei einer bestimmten Zugkraft die stoffschlüssige Verbindung zwischen Faden und Schutzhülle getrennt wird. Ein umständliches und aufwendiges Lösen des Fadens unter Verwendung der Fingernägel ist also nicht erforderlich, so daß bei der Anwendung des erfindungsgemäßen Tampons eine verbesserte Handhabung erzielt wird.

Somit bedarf es eines Ergreifens des Fadens - wie bei den im Stand der Technik bekannten Tampons üblich - nun nicht mehr, so daß zusätzlich die Gefahr einer Beschädigung der Oberfläche des Tampons, insbesondere das Herauslösen von Faserverbänden, beispielsweise durch die Fingernägel, vermieden wird. Auf diese Weise kann ein schädliches Verbleiben vom Tampon abgelöster Faserverbände nach der Benutzung in der Körperhöhle ausgeschlossen werden.

Um eine einfache und zuverlässige Verbindung des aus dem Grundkörper herausgeführten Teils des Fadens mit dem die hintere Stirnfläche abdeckenden Teil der Schutzhülle zu erreichen, wird in vorteilhafter Ausgestaltung der Erfindung vorgeschlagen, daß der die hintere Stirnfläche abdeckende Teil der Schutzhülle durch thermisches Verschweißen mit dem an diesem anliegenden Teilstück des Fadens verbunden ist. Gemäß einem weiteren Merkmal der Erfindung ist zum Entfernen der Schutzhülle ein Aufreißband vorgesehen, das vorzugsweise um den Umfang des Tampons herum in etwa auf Höhe der halben Länge des Tampons angeordnet ist. Dies bietet den Vorteil, daß nach Aufreißen der Schutzhülle mittels des Aufreißbandes diese in zwei im wesentlichen hülsenförmige Abschnitte aufgeteilt ist, von denen der eine das vordere Ende und der andere das diesem gegenüberliegende hintere Ende des Tampons umschließt. Durch axiales Auseinanderziehen dieser beiden Abschnitte, wodurch gleichzeitig der mit dem hinteren Abschnitt verbundene, aus dem Grundkörper herausgeführte Teil des Fadens langgestreckt wird, läßt sich dann die Schutzhülle von dem Tampon entfernen, ohne daß dieser Gefahr läuft, beschädigt zu werden.

Verfahrensmäßig wird die obige Aufgabe bei einem Verfahren zum Verpacken eines Tampons, der von im wesentlichen zylindrischer Gestalt ist, mit einem in Einführrichtung vorderen Ende, welches abgerundet oder kegelig geformt ist, sowie einem hinteren Ende mit einer Stirnfläche, aus der ein mit einem Teil seiner Länge in dem Tampon befestigter Faden oder Bändchen herausgeführt ist, erfindungsgemäß durch folgende Verfahrensschritte gelöst:
a) lockeres Fixieren des aus dem Tampon herausgeführten Teils des Fadens im Bereich der hinteren Stirnfläche an dem Tampon zur Sicherung der Lage des Fadens,
b) vollständiges Umschließen des Tampons einschließlich des locker fixierten Fadens mit einer Schutzhülle und
c) stoffschlüssiges Verbinden des die hintere Stirnfläche des Tampons abdeckenden Teils der Schutzhülle mit einem an diesem anliegenden Teilstück des Fadens.

Mit einem solchen Verfahren läßt sich der erfindungsgemäße Tampon fertigen. Ursächlich hierfür ist vor allem, daß der aus dem Tampon herausgeführte Teil des Fadens im Bereich der hinteren Stirnfläche zur Sicherung der Lage des Fadens locker fixiert und anschließend der die hintere Stirnfläche des Tampons abdeckende Teil der Schutzhülle stoffschlüssig mit einem an diesem anliegenden Teilstück des Fadens verbunden wird. Auf diese Weise wird beim Abziehen der Schutzhülle der aus dem Grundkörper herausgeführte Teil des Fadens gleichzeitig gestreckt und von der rückwärtigen Tamponstirnseite gelöst.

Von besonderem Vorteil ist es, wenn der die hintere Stirnfläche des Tampons abdeckende Teil der Schutzhülle mit dem an diesen anliegenden Teilstück des Fadens durch thermisches Verschweißen mittels einer der hinteren Stirnfläche gegenüberliegenden Hitzequelle verbunden wird. Dies gewährleistet eine sichere Befestigung des Fadens an der Schutzhülle während des Abziehens derselben, insbesondere solange, bis der Faden langgestreckt ist und aufgrund der dann auftretenden Zugbeanspruchung von der Schutzhülle getrennt wird. Durch diese Anordnung der Hitzequelle ist ein punktuelles thermisches Verschweißen sichergestellt, ohne daß andere Bereiche der Schutzhülle beeinträchtigt werden. Ein weiterer Vorteil ergibt sich daraus, daß durch das Verschweißen von Schutzhülle und Faden gleichzeitig die Schutzhülle weitgehend luft- und feuchtigkeitsdicht verschlossen wird.

Hinsichtlich einer vorteilhaften Verfahrensführung wird ferner vorgeschlagen, daß die Einwirkungsdauer der Hitzequelle 0,2 s bis 1,5 s beträgt bei einer Temperatur des verwendeten Heizelements zwischen 80°C und 250°C.

Um eine besonders einfache Handhabung des Tampons zu gewährleisten, wird schließlich vorgeschlagen, die Schutzhülle mit einem Aufreißband zu versehen.

Weitere Einzelheiten und Vorteile der Gegenstände der Erfindung ergeben sich aus der nachfolgenden Beschreibung eines bevorzugten Ausführungsbeispieles, das in der Zeichnung dargestellt ist, und zwar zeigen im einzelnen:
- Fig. 1: eine schematische Darstellung des Verschweißens der Schutzhülle eines Tampons mittels einer dessen hinterer Stirnfläche gegenüberliegenden Hitzequelle;
- Fig. 2: eine Seitenansicht eines in eine Schutzhülle eingeschweißten Tampons mit geschlossenem Aufreißband;
- Fig. 2a: eine Ansicht des unverdeckten hinteren Endes des Tampons gemäß Fig. 2;
- Fig. 3: eine Seitenansicht des Tampons gemäß Fig. 2 mit entferntem Aufreißband;
- Fig. 3a: eine Ansicht des durch die Schuzthülle verdeckten hinteren Endes des Tampons gemäß Fig. 2;
- Fig. 4: eine Seitenansicht des Tampons gemäß Fig. 3 mit auseinandergezogener Schutzhülle und
- Fig. 5: eine Seitenansicht des Tampons gemäß Fig. 4 mit vollständig entfernter Schutzhülle und sich frei erstreckendem Faden.

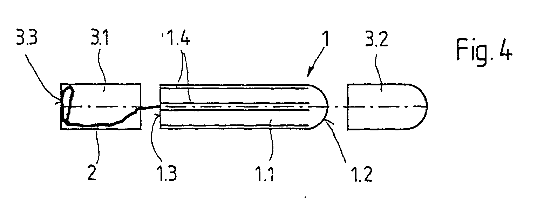

Der in den Fig. 1 bis 5 zu erkennende Tampon 1 besteht aus einem im wesentlichen zylindrischen Grundkörper 1.1, der mit einem in Einführrichtung vorderen, abgerundeten oder kegeligen Ende 1.2 und einer hinteren planen Stirnfläche 1.3 versehen ist. Aus der hinteren Stirnfläche 1.3 ist ein in dem Grundkörper 1.1 befestigter Faden 2 herausgeführt, wie insbesondere den Fig. 4 und 5 zu entnehmen ist. Während der Tampon 1 aus einem in die zuvor beschriebene Form gepreßten Faservliesmaterial besteht, ist der Faden 2 aus Baumwolle oder Zellwolle gefertigt. Der Grundkörper 1.1 ist zudem mit sich in Längsrichtung erstreckenden Rippen 1.4 versehen.

Der in den Fig. 1 bis 3 dargestellte Tampon 1 ist von einer Schutzhülle 3 umschlossen, die sich aus einem annähernd hülsenförmigen hinteren Abschnitt 3.1, der das hintere Ende mit der Stirnfläche 1.3 des Tampons 1 umschließt, und einem annähernd hülsenförmigen vorderen Abschnitt 3.2, der das vordere Ende 1.2 des Tampons 1 umschließt, zusammensetzt. In etwa auf Höhe der halben Länge des Tampons 1 ist an der Innenseite der Schutzhülle 3 ein Aufreißband 4 um den Umfang des Tampons 1 herum angeordnet. Die Schutzhülle 3 sowie das Aufreißband 4 sind aus einer glasklaren Folie, beispielsweise Cellophan gefertigt. Alternativ kann die Schutzhülle 3 und das Aufreißband 4 auch aus Polypropylen bestehen.

Zum Verpacken des Tampons 1 mit der Schutzhülle 3 wird als erstes der aus dem Grundkörper 1.1 herausgeführte Teil des Fadens 2 locker im Bereich der hinteren Stirnfläche 1.3 des Tampons 1 fixiert, wie insbesondere in den Fig. 2a und 3a ersichtlich. Anschließend wird der Tampon 1 einschließlich des locker fixierten Fadens 2 vollständig von der Schutzhülle 3 umschlossen. Zum Schluß wird der die hintere Stirnfläche 1.3 des Tampons 1 abdeckende Teil 3.3 des hinteren Abschnitts 3.1 der Schutzhülle 3 verschweißt und auf diese Weise gleichzeitig mit einem an diesem anliegenden Teilstück des Fadens 2 verbunden.

Das thermische Verschweißen des abdeckenden Teils 3.3 zum Schließen der Schutzhülle 3 und zum Verbinden mit dem Faden 2 ist in Fig. 1 dargestellt. Zu erkennen ist, daß sich der Tampon 1 dabei in einer sacklochartigen Aufnahme 5 befindet, die den Tampon 1 bis auf den Bereich der hinteren Stirnfläche 1.3 vollständig umschließt. Der hinteren Stirnfläche 1.3 gegenüberliegend ist ein Heizelement 6 anordbar, wie durch den Doppelpfeil in Fig. 1 angedeutet ist, mit dem punktuell der die hintere Stirnfläche 1.3 des Tampons abdeckende Teil 3.3 der Schutzhülle 3 erwärmt wird. Bei einer Einwirkungsdauer von 0,2 s bis 1,5 s beträgt hierfür die Temperatur zwischen 80°C und 250°C.

In den Fig. 2 und 2a bzw. 3 und 3a ist der auf zuvor beschriebene Weise mit der Schutzhülle 3 versehene Tampon 1 gezeigt. Im in Fig. 2 erkennbaren Zustand befindet sich das Aufreißband 4 noch unter der Schutzhülle 3 zwischen dem vorderen Abschnitt 3.2 und hinteren Abschnitt 3.1 der Schutzhülle 3, so daß der Tampon 1 weitgehend luft- und feuchtigkeitsgeschützt versiegelt ist. Im in Fig. 3 zu erkennenden Zustand ist das Aufreißband 4 bereits entfernt. Der vordere Abschnitt 3.2 sowie der hintere Abschnitt 3.1 der Schutzhülle 3 lassen sich nun in entgegengesetzter axialer Richtung von dem Tampon 1 abziehen, wie in Fig. 4 ersichtlich. Vorteilhafterweise wird zuerst der hintere Abschnitt 3.1 der Schutzhülle 3 abgezogen und währenddessen der Tampon 1 an dem vorderen Abschnitt 3.2 der Schutzhülle 3 gehalten. Auf diese Weise wird ein vorzeitiges Berühren des Tampons 1 mit den Fingern vermieden, wodurch der Tampon 1 vor Beschädigung oder Verunreinigung geschützt bleibt. Hierbei kommt auch zum Tragen, daß beim Abziehen des hinteren Abschnitts 3.1 der Schutzhülle 3 der die hintere Stirnfläche 1.3 des Tampons 1 abdeckende Teil 3.3 der Schutzhülle 3, der mit dem an diesem anliegenden Teilstück des Fadens 2 verbunden ist, diesen streckt, so daß der Faden 2 vom Tampon 1 losgelöst wird. Ist der Faden 2 vollständig gestreckt, so wird die Verbindung zwischen Faden 2 und dem Teil 3.3 der Schutzhülle 3 aufgrund der dann auftretenden Zugspannung getrennt, so daß sich der Faden - wie insbesondere in Fig. 5 erkennbar - frei erstrecken kann.

Mit dem zuvor beschriebenen Tampon sowie Verfahren zum Verpacken desselben wird eine bei der Anwendung verbesserte Handhabung erreicht. Indem ein Teilstück des Fadens 2 mit der Schutzhülle 3 verbunden ist, wird gleichzeitig mit Entfernen der Schutzhülle 3 der aus dem Grundkörper 1.1 herausgeführte Teil des Fadens 2 von dem Tampon 1 gelöst. Ein umständliches und aufwendiges manuelles Lösen des Fadens 2 von dem Tampon 1 wird dadurch hinfällig. Nicht zuletzt läßt sich auf diese Weise der Tampon 1 vor Beschädigungen schützen, da keine Faserverbände herausgelöst werden.

### Bezugszeichenliste

- 1: Tampon
- 1.1: Grundkörper
- 1.2: vorderes Ende
- 1.3: hintere Stirnfläche
- 1.4: Rippe
- 2: Faden
- 3: Schutzhülle
- 3.1: hinterer Abschnitt
- 3.2: vorderer Abschnitt
- 3.3: abdeckende Teil
- 4: Aufreißband
- 5: Aufnahme
- 6: Heizelement

## Patentansprüche

1. Tampon (1), insbesondere für die Frauenhygiene, bestehend aus einem im wesentlichen zylindrischen Grundkörper (1.1) mit einem in Einführrichtung vorderen Ende (1.2), das abgerundet oder kegelig geformt ist, sowie einem hinteren Ende mit einer Stirnfläche (1.3), aus der ein mit einem Teil seiner Länge in dem Grundkörper (1.1) befestigter Faden (2) oder ein Bändchen herausgeführt ist, wobei Grundkörper (1.1) und Faden (2) von einer zur Benutzung des Tampons (1) entfernbaren Schutzhülle (3) umschlossen sind,
**dadurch gekennzeichnet,**
**daß** der aus dem Grundkörper (1.1) herausgeführte Teil des Fadens (2) bei von der Schutzhülle (3) umschlossenem Tampon (1) vollständig im Bereich der hinteren Stirnfläche (1.3) angeordnet ist, wobei ein an dem die hintere Stirnfläche (1.3) des Tampons abdeckenden Teil (3.3) der Schutzhülle (3) anliegendes Teilstück des Fadens (2) durch thermisches Verschweißen mit der Schutzhülle (3) verbunden ist.

2. Tampon nach Anspruch 1, **dadurch gekennzeichnet, daß** zum Entfernen der Schutzhülle (3) ein Aufreißband (4) vorgesehen ist, das vorzugsweise um den Umfang des Tampon (1) herum auf Höhe der halben Länge des Tampon (1) angeordnet ist.

3. Verfahren zum Verpacken eines Tampons (1), der von im wesentlichen zylindrischer Gestalt ist, mit einem in Einführrichtung vorderen Ende (1.2), welches abgerundet oder kegelig geformt ist, sowie einem hinteren Ende mit einer Stirnfläche (1.3), aus der ein mit einem Teil seiner Länge in dem Tampon (1) befestigter Faden (2) oder Bändchen herausgeführt ist, mit den folgenden Schritten:
a) lockeres Fixieren des aus dem Tampon (1) herausgeführten Teils des Fadens (2) im Bereich der hinteren Stirnfläche (1.3) an dem Tampon (1) zur Sicherung der Lage des Fadens (2),
b) vollständiges Umschließen des Tampons (1) einschließlich des locker fixierten Fadens (2) mit einer Schutzhülle (3) und
c) stoffschlüssiges Verbinden des die hintere Stirnfläche (1.3) des Tampons (1) abdeckenden Teils (3.3) der Schutzhülle (3) mit einem an diesem anliegenden Teilstück des Fadens (2).

4. Verfahren nach Anspruch 3, **dadurch gekennzeichnet, daß** der die hintere Stirnfläche (1.3) des Tampons (1) abdeckende Teil (3.3) der Schutzhülle (3) mit dem an diesen anliegenden Teilstück des Fadens (2) durch thermisches Verschweißen mittels einer der hinteren Stirnfläche (1.3) gegenüberliegenden Hitzequelle (6) verbunden wird.

5. Verfahren nach Anspruch 4, **dadurch gekennzeichnet, daß** die Einwirkungsdauer der Hitzequelle (6) 0,2 s bis 1,5 s bei einer Temperatur des verwendeten Heizelementes (6) zwischen 80°C und 250°C beträgt.

6. Verfahren nach einem der Ansprüche 3 bis 5, **dadurch gekennzeichnet, daß** die Schutzhülle (3) mit einem Aufreißband (4) versehen wird.

## Claims

1. Tampon (1), in particular for feminine hygiene, consisting of an essentially cylindrical base body (1.1) having, in the insertion direction, a front end (1.2) which is rounded or cone-shaped and a rear end with an end face (1.3) from which there protrudes a string (2) or small strip which is fastened by part of its length in the base body (1.1), wherein base body (1.1) and string (2) are enclosed by a protective sleeve (3) which can be removed in order to use the tampon (1), **characterized in that** the part of the string (2) which protrudes from the base body (1.1) is completely arranged in the region of the rear end face (1.3) when the tampon (1) is enclosed by the protective sleeve (3), wherein a part of the string (2) which adjoins the part (3.3) of the protective sleeve (3) that covers the rear end face (1.3) of the tampon is connected to the protective sleeve (3) by thermal bonding.

2. Tampon according to Claim 1, **characterized in that** a tear strip (4) is provided for removing the protective sleeve (3), said tear strip preferably being arranged around the circumference of the tampon (1) half-way along the length of the tampon (1).

3. Method of packaging a tampon (1) which is of essentially cylindrical shape having, in the insertion direction, a front end (1.2) which is rounded or cone-shaped and a rear end with an end face (1.3) from which there protrudes a string (2) or small strip which is fastened by part of its length in the tampon (1), said method comprising the following steps:
a) loosely fixing the part of the string (2) which protrudes from the tampon (1) in the region of the rear end face (1.3) to the tampon (1) in order to secure the position of the string (2),
b) completely enclosing the tampon (1) including the loosely fixed string (2) by a protective sleeve (3), and
c) materially connecting the part (3.3) of the protective sleeve (3) which covers the rear end face (1.3) of the tampon (1) to a part of the string (2) which adjoins said protective sleeve.

4. Method according to Claim 3, **characterized in that** the part (3.3) of the protective sleeve (3) which covers the rear end face (1.3) of the tampon (1) is connected to the part of the string (2) which adjoins said protective sleeve by thermal bonding by means of a heat source (6) located opposite the rear end face (1.3).

5. Method according to Claim 4, **characterized in that** the duration of exposure to the heat source (6) is 0.2 s to 1.5 s at a temperature of the heating element (6) used of between 80°C and 250°C.

6. Method according to any of Claims 3 to 5, **characterized in that** the protective sleeve (3) is provided with a tear strip (4).

## Revendications

1. Tampon (1), en particulier pour l'hygiène féminine, constitué d'un corps de base (1.1) sensiblement cylindrique avec une extrémité avant (1.2) dans le sens d'introduction qui a une forme arrondie ou conique ainsi qu'une extrémité arrière avec une surface frontale (1.3) hors de laquelle sort un fil (2) fixé sur une partie de sa longueur dans le corps de base (1.1) ou une bandelette, le corps de base (1.1) et le fil (2) étant entourés d'une enveloppe de protection (3) amovible pour l'utilisation du tampon (1), **caractérisé en ce que** la partie du fil (2) sortant du corps de base (1.1) dans le tampon (1) entouré par l'enveloppe de protection (3) se trouve entièrement dans la zone de la surface frontale arrière (1.3), moyennant quoi une portion du fil (2) s'appuyant sur la partie (3.3) de l'enveloppe de protection (3) recouvrant la surface frontale arrière (1.3) du tampon est reliée à l'enveloppe de protection (3) par soudure thermique.

2. Tampon selon la revendication 1, **caractérisé en ce que**, pour enlever l'enveloppe de protection (3), il est prévu une bande d'arrachage (4) qui est placée, de préférence, autour de la circonférence du tampon (1) à la hauteur de la demi-longueur du tampon (1).

3. Procédé destiné à conditionner un tampon (1), qui a une forme sensiblement cylindrique, avec une extrémité avant (1.2) dans le sens d'introduction qui a une forme arrondie ou conique ainsi qu'une extrémité arrière avec une surface frontale (1.3) hors de laquelle sort un fil (2) ou une bandelette fixé sur une partie de sa longueur dans le tampon (1), avec les étapes suivantes :
a) fixation lâche de la partie du fil (2) sortant du tampon (1) dans la zone de la surface frontale arrière (1.3) au tampon (1) pour maintenir la position du fil (2),
b) encapsulation complète du tampon (1) y compris du fil (2) fixé de manière lâche dans une enveloppe de protection (3) et
c) liaison solidaire avec la matière de la partie (3.3) recouvrant la surface frontale arrière (1.3) du tampon (1) avec une portion du fil (2) s'appuyant sur celle-ci.

4. Procédé selon la revendication 3, **caractérisé en ce que** la partie (3.3) de l'enveloppe de protection (3) recouvrant la surface frontale arrière (1.3) du tampon (1) est reliée à la portion du fil (2) s'appuyant sur celle-ci par soudure thermique à l'aide d'une source de chaleur (6) opposée à la surface frontale arrière (1.3).

5. Procédé selon la revendication 4, **caractérisé en ce que** la durée d'effet de la source de chaleur (6) est comprise entre 0, 2 s et 1,5 s à une température de l'élément chauffant utilisé (6) comprise entre 80°C et 250°C.

6. Procédé selon l'une quelconque des revendications 3 à 5, **caractérisé en ce que** l'enveloppe de protection (3) est munie d'une bande d'arrachage (4).
